# EUROPEAN PATENT SPECIFICATION

(11) **EP 3 294 370 B1**
(45) Date of publication and mention of the grant of the patent: **10.04.2024**
(21) Application number: 16721915.3
(22) Date of filing: 09.05.2016
(51) Int. Cl.: A61M 1/36, A61M 25/00, A61M 16/04

(54) **A CONDUIT ARRANGEMENT**
LEITUNGSANORDNUNG
AGENCEMENT DE CONDUIT

(30) Priority: 08.05.2015 GB 201507919
(43) Date of publication of application: 21.03.2018
(73) Proprietor: Vascular Flow Technologies Limited, Dundee DD2 1TY (GB)
(72) Inventor: HOUSTON, John Graeme, Perth PH2 7AW (GB); ALLAN, William David, Hemingford Grey Cambridgeshire PE28 9BJ (GB); DUNLOP, Craig Maxwell, Glasgow G77 5SU (GB)
(74) Representative: Ouzman, Beverley Nicola Claire
(86) International application number: PCT/GB2016/051324
(87) International publication number: WO 2016/181121

(56) References cited:
- EP-A1- 0 175 595
- EP-A1- 0 386 408
- EP-A1- 2 559 450
- WO-A1-2007/110074
- WO-A2-00/38591
- CN-U- 204 293 665
- US-A- 4 681 570
- US-A1- 2002 198 432
- US-A1- 2005 277 862

## Description

### Field of invention

The present invention relates to an intravenous dialysis catheter and, more particularly, an intravenous dialysis catheter comprising two tubular lumens for the independent transfer of fluid.

### Background of the invention

In the field of medicine, it is necessary for fluids essential to life, such as blood, to be transferred independently and, more particularly, to be transferred independently to and from an individual. For example, individuals suffering from kidney failure require regular dialysis treatment. Such dialysis treatment requires the removal of blood from the individual and the cycling of the blood through a dialysis machine that performs the function of the failed kidney. After processing, the blood is then returned to the individual. A dialysis catheter is used to transfer blood to and from an individual during such dialysis treatment. A dialysis catheter comprises a dual lumen to keep separate the blood flowing out of the individual and into the dialysis machine from that flowing out of the dialysis machine and into the individual. One lumen of the dialysis catheter is an outflow lumen, which transfers outgoing blood from the dialysis machine to the individual and the other lumen is an inflow lumen, which transfers incoming blood from the individual to the dialysis machine. A dialysis catheter is typically inserted into a vein in the neck, chest or groin of an individual. A further example of the independent transfer of fluid to and from an individual is multiple lumen intravenous catheters; for example, central venous catheters for fluid administration.

WO00/38591 relates to artificial or modified natural blood flow tubing (also known as a "vascular graft"). The blood flow tubing of WO00/38591 seeks to replicate natural blood flow, which occurs in a spiral fashion throughout the body (Stonebridge PA, Brophy CM.,1991, Spiral laminar flow in arteries?; The Lancet. 338 (8779): pp1360-1). In particular, the blood flow tubing of WO00/38591 has helical-flow inducing means adapted to induce helical flow in such a fashion as to eliminate or reduce turbulence. The tubing has internal helical grooving and/or ridging which induces such helical flow. In a vascular graft, the provision of helical blood flow reduces the turbulence of the blood in the vascular graft which, in turn, reduces the likelihood of plaque formation, reduced flow capacity and thromboses.

CN204293665 relates to a three-cavity catheter comprising a main cavity coaxial with the axis of the catheter body and first and second secondary cavities which wrap the periphery of the main cavity. Two partition boards are arranged between the first and second secondary cavities and are spirally distributed along the axis of the catheter body. The first and second secondary cavities have a C-shaped cross-sectional profile. The spiral partition boards function to prevent the first and second secondary cavities from being stressed and deforming. Thus the design of CN204293665 is focused on providing support for the secondary cavities as opposed to seeking to improve flow characteristics. The problem with the three-cavity catheter reported in CN204293665 is that it does not produce spiral laminar flow owing to the C-shaped cross-sectional profiles of the secondary cavities. Therefore, natural blood flow patterns and the associated advantages are not attained by the three-cavity catheter of CN204293665.

EP 2559450 discloses a shaft for medical devices comprising an inner and outer tube. The shaft is for an inflation catheter and the spiral geometry allows the speed of deflation to be increased.

An entirely different technical field is that of devices for improving the quality of drinking water and other beverages. WO2007/110074 relates to an apparatus for treating a liquid such as water or wine in order to improve its quality. The apparatus comprises at least two pipe segments which are twisted about a common axis. The pipe segments comprise inlet and outlet openings which are connectable to a common inlet and outlet such that the flow of liquid is divided in the pipe segments and then reunited into a single flow of liquid which leaves the apparatus via a common outlet opening. As such, the problem with the apparatus of WO2007/110074 is that it is fundamentally unsuited for carrying bodily fluids such as blood. It is, furthermore, to be noted that the design of the apparatus comprises common inlet and outlet openings meaning that the flow of liquids in the different pipe segments is not kept separate. Instead, the liquid flowing through the different pipe segments is re-mixed.

The present invention seeks to alleviate the above problems and arises from the recognition that blood flow in a dialysis catheter and fluid flow in a multiple lumen intravenous catheter is not helical and that it would be desirable for it to be so.

In particular, it would be desirable for blood flow in the outflow lumen of the dialysis catheter to occur in a helical fashion such that blood entering the vein of the individual at the site of insertion of the dialysis catheter would replicate natural blood flow patterns. Likewise, it would be desirable to induce such properties in the fluid flowing in the lumen of multiple lumen intravenous catheters. In particular, it would be desirable for blood flow in the lumen of a dialysis catheter or in the lumen of a multiple lumen intravenous catheter to have spiral laminar flow (i.e. to replicate natural blood flow).

Additional advantages associated with helical fluid flow, in particular spiral laminar flow, in a dialysis catheter or in a multiple lumen intravenous catheter include: (a) the fluid, such as blood, is subjected to less shear stress when flowing in a helical fashion; and (b) higher volumes of fluid can be transferred in a conduit of a given diameter when the fluid has helical flow as compared to non-helical flow. For these reasons, it would be desirable to induce helical fluid flow, in particular spiral laminar flow, in a dialysis catheter or in a multiple lumen intravenous catheter.

There are also a range of clinical benefits to inducing helical fluid flow, such as helical blood flow, in particular spiral laminar flow, in a dialysis catheter or in a multiple lumen intravenous catheter. The helical fluid flow reduces turbulence in the catheter, which in turn: reduces the tendency of thrombosis in the catheter; reduces the tendency of infection in the catheter because there is reduced stagnation (stagnation may lead to infection and the formation of infected "biofilm" that makes infection eradication problematic); and reduces endothelial damage at the end of catheter, which reduces venous or vessel tissue response and narrowing. The helical fluid flow increases the velocity of fluid flowing in the lumen, which reduces stasis or stagnation and so reduces thrombosis. In addition, the helical fluid flow reduces stagnation and/or cellular deposition. If the intravenous catheter is used to transfer an infusate comprising a drug to an individual, such as during chemotherapy, the helical fluid flow improves mixing of the fluid contents in the lumen of the catheter. This affects the distribution of the drug in the infusate and may reduce the toxic level of a drug locally that could affect a vessel wall.

These clinical advantages are primarily associated with the induction of helical fluid flow, in particular spiral laminar flow, in the lumen of the catheter that transfers fluid to the individual. However, the induction of helical flow in the lumen of the catheter that transfers fluid away from the individual can also be of benefit; for example, to reduce shear stress on the fluid or to enable higher volumes of fluid to be transferred in a conduit of a given diameter.

### Summary of the Invention

In accordance with a first aspect of the invention, there is provided an intravenous dialysis catheter as defined in the claims.

The helical flow is spiral laminar flow.

Preferably, the portion of the tubular lumen capable of imparting helical flow is 10%, 20%, 25%, 30%, 40%, 50%, 60%, 70%, 75%, 80%, 90% or 100% of the total length of the tubular lumen.

The catheter comprises a tubular conduit and a septum which divides the tubular conduit parallel to its longitudinal axis so as to define the two tubular lumens.

Preferably, the septum extends substantially across a diameter of the tubular conduit.

Preferably, the pathway of each of the two tubular lumens is straight with respect to the longitudinal axis of each tubular lumen.

A portion of the tubular lumen comprises an axially extending internal helical protrusion located around the inside of the tubular lumen for imparting helical flow on fluid passing through said portion of the tubular lumen.

Optionally, a portion of the tubular lumen follows a helical pathway.

Optionally, sequential non-circular cross-sections of said portion of the tubular lumen transition rotationally along the longitudinal axis of the tubular lumen.

Advantageously, each of the two tubular lumens independently follows a helical pathway.

At least one of the tubular lumens comprises an axially extending internal helical protrusion located around the inside of the tubular lumen for imparting helical flow on fluid passing through the tubular lumen.

Advantageously, the conduit arrangement is made from polyurethane.

Alternatively, the conduit arrangement is made from PVC, PTFE, latex, silicone or carbothane.

Conveniently, the conduit arrangement is flexible.

Conveniently, the interior of each of the two tubular lumens is made from a biocompatible material.

The catheter of the invention, is useful in a method of transferring fluid.

Preferably, the fluid is transferred to and/or from an individual.

Conveniently, the fluid is blood.

The terms "helix" and "helix angle" as used herein cover the mathematical definitions of helix and helical and any combination of mathematical definitions of helical and spiral.

A "helix angle" referred to herein is the angle between the helix and the axial line about which it is formed, in particular the longitudinal axis of a tubular lumen.

The term "outflow lumen" as used herein refers to a tubular lumen that transfers outgoing blood from an external source, such as a dialysis machine, to an individual.

The term "inflow lumen" as used herein refers to a tubular lumen that transfers incoming blood from an individual to an external location, such as a dialysis machine. The term "spiral laminar flow" as used herein refers to a linear flow with a rotational vector flow component centred on the axis of the tubular conduit. In other words, in addition to the laminar flow in the direction of tubular conduit, there is a rotation in the plane of the cross-section of the tubular conduit.

The presence of spiral laminar flow can be determined using colour Doppler ultrasound *in vivo* as described by Stonebridge et al. in Clinical Science (1996, 9: 17-21) The presence of spiral laminar flow results in a characteristic "red/blue" split being seen using this technique. In addition, colour Doppler ultrasound can be used with a flow rig in order to determine the presence of spiral laminar flow *ex vivo.* For example, a flow rig comprising a fluid circuit and a blood mimic at 37°C having either a constant or pulsatile flow can be used to study blood flow characteristics.

In addition, the presence of spiral laminar flow can be determined using phase contrast magnetic resonance imaging (MRI). Houston et al. in Nephrol Dial Transplant. (2004 Jul;19(7):1786-91), describes the use of MRI to observe the prevalence of spiral laminar flow *in vivo.*

### Brief Description of the Figures

Figure 1 is a perspective view of a tubular conduit of a first embodiment, not in accordance with the present invention, showing views along each of the lines A-A, B-B and C-C.
Figure 2 is a perspective view of a tubular conduit, in accordance with a second embodiment of the present invention, showing views along each of the lines A'-A', B'-B' and C'-C.
Figure 3 is a perspective view of a portion of an intermediate conduit arrangement, in accordance with a third embodiment of the present invention.
Figure 4 is a perspective view of a portion of an intermediate conduit arrangement, in accordance with a variant of the third embodiment of the present invention.
Figure 5 is a perspective view of a portion of an intermediate conduit arrangement, in accordance with a further variant of the third embodiment of the present invention.
Figure 6 is a perspective view of an intermediate conduit arrangement, in accordance with a further variant of the third embodiment of the present invention.
Figure 7 is a perspective view of a conduit arrangement, in accordance with the third embodiment of the present invention.
Figure 8 is a perspective view of a portion of a tubular conduit, in accordance with a fourth embodiment of the present invention, with some hidden detail shown in dashed lines.
Figure 9A is a cross-sectional view of an outflow lumen of a tubular conduit through a plane perpendicular to the longitudinal axis of the tubular conduit.
Figure 9B is an enlarged section showing detail of the base of an axially extending internal helical protrusion as circled in Figure 9A.

### Detailed Description of the Invention

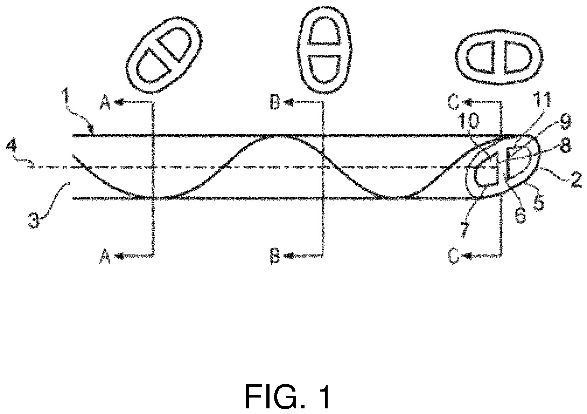

Referring to Figure 1, a tubular conduit 1 of a first embodiment not in accordance with the present invention is shown. The tubular conduit 1 comprises proximal and distal ends 2,3 and a longitudinal axis 4 therebetween. As can be seen from the views along each of the lines A-A, B-B and C-C, the tubular conduit 1 is defined by an axially extending, elliptical perimeter wall 5 which defines the exterior of the tubular conduit 1. Thus, the tubular conduit 1 has an elliptical cross-section. The tubular conduit further comprises an axially extending septum 6, which extends substantially across a diameter of the tubular conduit 1. The inner surface 7 of the perimeter wall 5 of the tubular conduit 1 and each side 8, 9 of the septum 6 define two axially extending tubular lumens: an inflow lumen 10 and an outflow lumen 11. Each of the inflow lumen 10 and the outflow lumen 11 are D-shaped in cross-section and each independently permits fluid communication between the proximal and distal ends 2, 3 of the tubular conduit 1.

In this embodiment, each of the inflow and outflow lumens 10, 11 follows a helical pathway, with a helix angle of 20°, between the proximal and distal ends 2, 3 of the tubular conduit 1 such that sequential non-circular cross-sections of the tubular conduit 1 transition rotationally along the length of the tubular conduit 1. The helical pathway of each of the inflow and outflow lumens 10, 11 between the proximal and distal ends 2, 3 of the tubular conduit 1 consists of one single revolution. That is to say, the pathway of each of the inflow and outflow lumens 10, 11 between the proximal and distal ends 2, 3 of the tubular conduit 1 makes one complete turn of 360°.

In use, the tubular conduit 1 is comprised within a dialysis catheter (not shown) and the distal end 3 of the tubular conduit 1 is connected so as to be in fluid communication with a dialysis machine (not shown) and the proximal end 2 of the tubular conduit 1 is inserted into the vein of an individual who is suffering from kidney failure (not shown). At the distal end 3 of the tubular conduit 1, the inflow and outflow lumens 10, 11 separate into two independent conduits (not shown) so as to permit independently fluid communication with a dialysis machine (not shown). At the proximal end 2 of the tubular conduit 1, the inflow and outflow lumens 10, 11 also separate into two independent conduits so as to prevent mixing of the blood in the vein of the individual (not shown). In alternative embodiments, the proximal end 2 of each of the inflow and outflow lumens 10, 11 is staggered with respect to the other so as to prevent mixing of the blood in the vein of the individual (not shown).

Once the distal end 3 of the tubular conduit 1 is connected so as to be in fluid communication with the dialysis machine and the proximal end 2 is inserted into the vein of the individual, blood flows independently from the individual to the dialysis machine through the inflow lumen 10 of the tubular conduit 1 and from the dialysis machine to the individual through the outflow lumen 11 of the tubular conduit 1. As the blood passes through each of the inflow and outflow lumens 10, 11, the helical pathway of said lumens imparts helical flow on the blood, which reduces turbulence in the blood. The helical blood flow continues after the blood exits the outflow lumen 11 and enters the blood vessel of the individual. Thus turbulent blood flow is reduced, or even eliminated, in the blood vessel downstream of the outflow lumen 11.

In a variant of the first embodiment, the inflow and outflow lumens 10, 11 each follow a helical pathway along only a portion of the length of the tubular conduit 1. In this variant of the first embodiment, the inflow and outflow lumens 10, 11 each follow a helical pathway, with a helix angle of 20°, between the proximal end 2 of the tubular conduit 1 and a termination point, which is short of the distal end 3 of the tubular conduit 1. In a further variant of the first embodiment, the inflow and outflow lumens 10, 11 each follow a helical pathway, with a helix angle of 20°, between the distal end 3 of the tubular conduit 1 and a termination point, which is short of the proximal end 2 of the tubular conduit 1. The helical pathway of each of the inflow and outflow lumens 10, 11 between the proximal end 2 of the tubular conduit 1 and the termination point or, alternatively, between the distal end 3 of the tubular conduit 1 and the termination point consists of one single revolution. That is to say, the pathway of each of the inflow and outflow lumens 10, 11 between the proximal end 2 of the tubular conduit 1 and the termination point or, alternatively, between the distal end 3 of the tubular conduit 1 and the termination point makes one complete turn of 360°. From the termination point to the distal end 3 of the tubular conduit or, alternatively, from the termination point to the proximal end 2 of the tubular conduit, the pathway of each of the inflow and outflow lumens 10, 11 is substantially straight with respect to the longitudinal axis 4 of the tubular conduit, subject to any overall curvature of the tubular conduit 1. It is to be appreciated that the total length of the tubular conduit 1 varies depending on its specific use. However, a dialysis catheter typically comprises a tubular conduit 1 that is between approximately 5 and 55 cm in length. In this variant, the proportion of the length of the tubular conduit 1 in which the inflow and outflow lumens 10, 11 follow a helical pathway varies but it is generally less than 50% of the total length of the tubular conduit 1. In preferred embodiments, the proportion of the length of the tubular conduit 1 in which the inflow and outflow lumens 10, 11 follow a helical pathway is less than 25% or less than 15% of the total length of the tubular conduit 1.

In a further variant of the first embodiment, the length of the tubular conduit 1 in which the inflow and outflow lumens 10, 11 follow a helical pathway is selected with reference to the diameter of the lumen of the tubular conduit 1. This relationship is explained in Table 1 below. For example, for a tubular conduit with a lumen diameter of 2mm, the minimum length of the tubular conduit in which the inflow and outflow lumens 10, 11 follow a helical pathway would be 17.26mm. In embodiments such as the first embodiment, where each lumen 10, 11 of the tubular conduit is D-shaped, the diameter of the lumen is taken as an approximation or average of the diameter of the D-shaped lumen.

**Table 1: Relationship between lumen diameter and minimum length of helical flow inducing means**

| **Lumen Diameter (mm)** | **Minimum length of helical flow inducing means (mm)** |
|---|---|
| 1 | 8.63 |
| 2 | 17.26 |
| 3 | 25.89 |
| 4 | 34.53 |
| 5 | 43.16 |
| 6 | 51.79 |
| 7 | 60.42 |
| 8 | 69.05 |
| 9 | 77.68 |
| 10 | 86.31 |
| 11 | 94.95 |
| 12 | 103.58 |
| 13 | 112.21 |
| 14 | 120.84 |
| 15 | 129.47 |
| 16 | 138.10 |
| 1 7 | 146.73 |
| 18 | 155.37 |
| 19 | 164.00 |
| 20 | 172.63 |

In the first embodiment and the variants of the first embodiment described above, the helical pathway of each of the inflow and outflow lumens 10, 11 consists of one single revolution. However, in alternative embodiments, the helical pathway of each of the inflow and outflow lumens 10, 11 is either shorter or longer than one single revolution and is, for example, between 50% and 150% of a single revolution.

In use, the variants of the first embodiment operate in substantially the same way as the first embodiment. As blood passes through each of the inflow and outflow lumens 10, 11 in the portion of the length of the tubular conduit 1 that follows a helical pathway, helical flow is imparted on the blood which reduces turbulence in the blood. Furthermore, the helical flow of the blood in the inflow or outflow lumens 10, 11 continues after the blood passes the termination point of the helical pathway. Therefore, turbulent blood flow is likewise reduced, or even eliminated, along the length of the inflow or outflow lumen 10, 11. Similarly, the helical blood flow continues after the blood exits the outflow lumen 11 at the proximal end 2 of the tubular conduit 1 and enters the blood vessel of the individual. Thus turbulent blood flow is reduced, or even eliminated, in the blood vessel downstream of the outflow lumen 11.

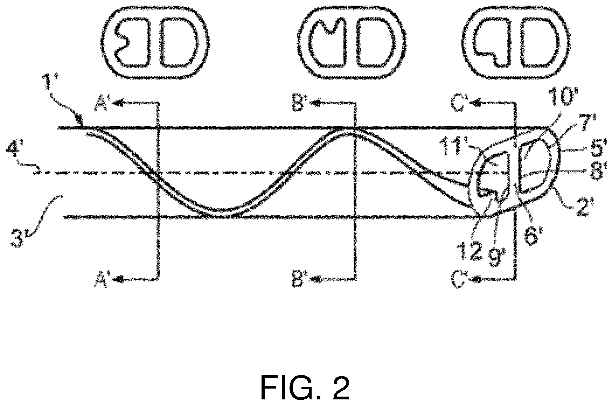

Referring to Figure 2, a tubular conduit 1' in accordance with a second embodiment of the present invention is shown. The tubular conduit 1' comprises proximal and distal ends 2', 3' and a longitudinal axis 4' therebetween. As can be seen from the views along each of the lines A'-A', B'-B' and C'-C', the tubular conduit 1' is defined by an axially extending, elliptical perimeter wall 5' which defines the exterior of the tubular conduit 1'. Thus, the tubular conduit 1' has an elliptical cross-section. The tubular conduit 1' further comprises an axially extending septum 6', which extends substantially across a diameter of the tubular conduit. The inner surface 7' of the perimeter wall 5' of the tubular conduit 1' and each side 8', 9' of the septum 6' define two axially extending tubular lumens 10', 11': an inflow lumen 10' and an outflow lumen 11'. Each of the inflow lumen 10' and the outflow lumen 11' is D-shaped in cross-section and each independently permits fluid communication between the proximal and distal ends 2', 3' of the tubular conduit 1'. Located around the inner surface 7' of the perimeter wall 5' and the side 9' of the septum 6' is an axially extending internal helical protrusion 12 which projects radially inwardly into the outflow lumen 11'. The helix angle of the axially extending internal helical protrusion 12 is 20°. In this embodiment, the cross- section of the axially extending internal helical protrusion 12 perpendicular to the longitudinal axis 4' of the tubular conduit 1' is of a bell shape. Sequential views along each of the lines A'-A', B'-B' and C'-C' demonstrate the location of the axially extending internal helical protrusion 12 along the length of the tubular conduit 1'. In this embodiment, the axially extending internal helical protrusion 12 consists of one single revolution. That is to say, the axially extending internal helical protrusion makes one complete turn of 360° between the proximal and distal ends 2', 3' of the tubular conduit 1'.

In use, the tubular conduit 1' is comprised within a dialysis catheter (not shown) and the distal end 3' of the tubular conduit 1' of the second embodiment is connected so as to be in fluid communication with a dialysis machine (not shown) and the proximal end 2' of the tubular conduit 1' is inserted into the vein of an individual who is suffering from kidney failure (not shown). The structure of the proximal and distal ends 2', 3' of the tubular conduit 1' can be the same as that described above in relation to the first embodiment.

Once the distal end 3' of the tubular conduit 1' is connected so as to be in fluid communication with the dialysis machine and the proximal end 2' is inserted into the vein of the individual, blood flows independently from the individual to the dialysis machine through the inflow lumen 10' of the tubular conduit 1' and from the dialysis machine to the individual through the outflow lumen 11 ' of the tubular conduit 1'. As the blood passes the axially extending internal helical protrusion 12 of the outflow lumen 11', helical flow is imparted on the blood, which reduces turbulence in the blood. Furthermore, the helical blood flow continues after the blood exits the outflow lumen 11' and enters the blood vessel of the individual. Thus turbulent blood flow is reduced, or even eliminated, in the blood vessel downstream of the outflow lumen 11'.

In a variant of the second embodiment, the axially extending internal helical protrusion 12 extends for only a portion of the length of the tubular conduit 1'. In this variant of the second embodiment, the axially extending internal helical protrusion 12 extends around the inner surface 7' of the wall 5' and the side 9' of the septum 6' from the proximal end 2' of the tubular conduit 1' to a termination point, which is short of the distal end 3' of the tubular conduit 1'. In a further variant of the first embodiment, the axially extending internal helical protrusion 12 extends around the inner surface 7' of the wall 5' and the side 9' of the septum 6' from the distal end 3' of the tubular conduit 1' to a termination point, which is short of the proximal end 2' of the tubular conduit 1'. The helix angle of the axially extending internal helical protrusion 12 is 20°. The axially extending internal helical protrusion 12 consists of one single revolution. That is to say, the axially extending internal helical protrusion 12 makes one complete turn of 360° between the proximal and distal ends 2', 3' of the tubular conduit 1'. It is to be appreciated that the total length of the tubular conduit 1' varies depending on its specific use. However, a dialysis catheter typically comprises a tubular conduit 1' that is between approximately 5 and 55 cm in length. The proportion of the length of the tubular conduit 1' which comprises an axially extending internal helical protrusion 12 varies but it is generally less than 50% of the total length of the tubular conduit 1'. In preferred embodiments, the proportion of the length of the tubular conduit 1' which comprises the axially extending internal helical protrusion 12 is less than 25% or less than 15% of the total length of the tubular conduit 1.

In a further variant of the second embodiment, the length of the axially extending internal helical protrusion 12 is selected with reference to the diameter of the lumen of the tubular conduit 1', as discussed above with reference to the first embodiment and Table 1.

In the variants of the second embodiment described above, the axially extending internal helical protrusion 12 consists of one single revolution. However, in alternative variants, the axially extending internal helical protrusion is either shorter or longer than one single revolution and may, for example, be between 50% and 150% of a single revolution.

In use, the variants of the second embodiment operate in substantially the same manner as is described for the second embodiment. As the blood passes through the outflow lumen 11' in the portion of the tubular conduit 1' that comprises the axially extending internal helical protrusion 12, helical flow is imparted on the blood which reduces turbulence in the blood. Furthermore, the helical blood flow continues after the blood passes the termination point of the axially extending internal helical protrusion 12. Therefore, turbulent flow is reduced, or even eliminated, along the length of the outflow lumen 11' that is downstream of the termination point. Similarly, the helical blood flow continues after the blood exits the outflow lumen 11' at the proximal end of the tubular conduit 1' and enters the blood vessel of the individual. Thus turbulent blood flow is reduced, or even eliminated, in the blood vessel downstream of the outflow lumen 11'.

In the first and second embodiments, the tubular conduit 1, 1' has an elliptical cross-section. However, in alternative embodiments, the tubular conduit 1, 1' has a circular cross-section or a substantially circular cross section.

In the first and second embodiments, the septum 6, 6' extends substantially across a diameter of the tubular conduit 1, 1'. However, in alternative embodiments, the septum 6, 6' follows a curved or an S-shaped path perpendicular to the longitudinal axis 4, 4' of the tubular conduit 1, 1'.

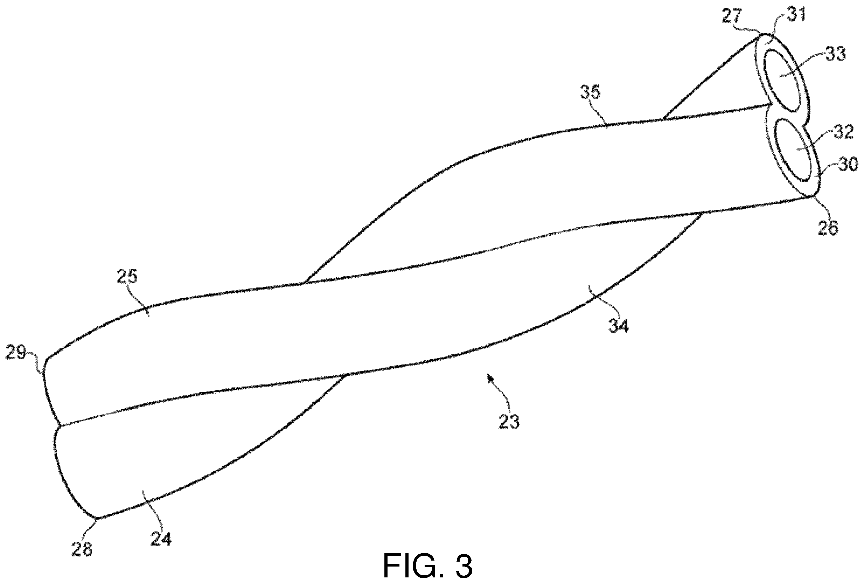

Referring to Figure 3, a portion of a conduit arrangement 23 in intermediate form for use in accordance with a third embodiment of the present invention is shown. The conduit arrangement 23 comprises first and second tubular conduits 24, 25, which each comprise proximal 26, 27 and distal 28, 29 ends and a tubular perimeter wall 30, 31 that extends therebetween. The tubular perimeter wall 30, 31 defines the exterior of the first and second tubular conduits 24, 25. Thus, the first and second tubular conduits have circular cross-sections. The tubular perimeter wall 30, 31 of the first and second tubular conduits 24, 25 further defines first and second tubular lumens 32, 33: an inflow lumen 32 and an outflow lumen 33. Each of the inflow and outflow lumens 32, 33 are circular in cross-section and each independently permits fluid communication between the proximal 26, 27 and distal 28, 29 ends of the tubular conduits 24, 25.

The first and second tubular conduits 24, 25 are intertwined such that each of the inflow and outflow lumens 32, 33 independently follows a helical pathway, having a helix angle of 20°, between the proximal 26, 27 and distal 28, 29 ends of the tubular conduits 24, 25. In some embodiments, the outer surface 34, 35 of the tubular perimeter wall 30, 31 of the first and second tubular conduits 24, 25 is fused together along the length of the first and second tubular conduits 24, 25 for stabilising the conduit arrangement 23. In other embodiments, at least a portion of the outer surface 34, 35 of the tubular perimeter wall 30, 31 of the first and second tubular conduits 24, 25 is fused together at intersection points (not shown) for stabilising the conduit arrangement 23.

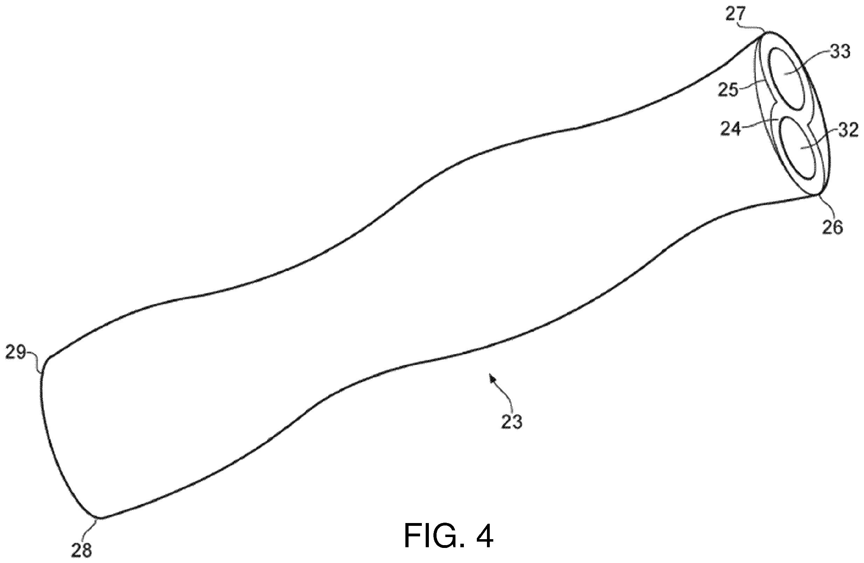

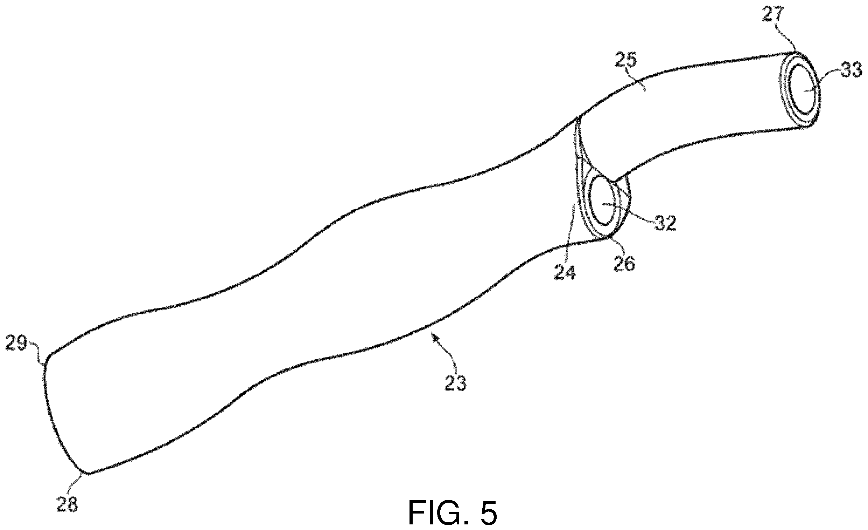

In a variant of the third embodiment, the conduit arrangement 23 in intermediate form is stabilised by embedding the first and second tubular conduits 24, 25 within a single, relatively larger tubular structure, as shown in Figure 4. Referring to Figure 5, a further variant is shown in which the proximal end 27 of the second tubular conduit 25 extends beyond the proximal end 26 of the first tubular conduit 24. Thus the outflow lumen 33 extends beyond the inflow lumen 32 at the proximal end 26, 27 of the conduit arrangement 23.

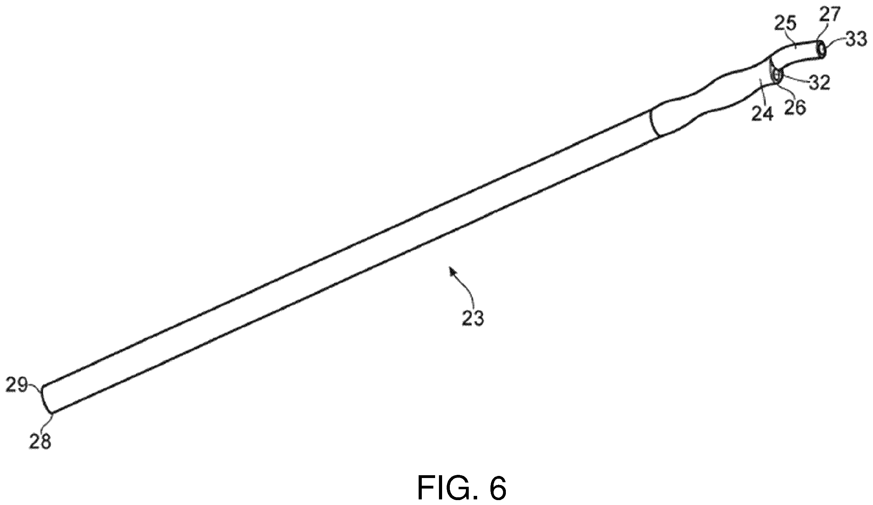

In a further variant, the inflow and outflow lumens 32, 33 independently follow a helical pathway along only a portion of the conduit arrangement 23. An example of such a variant is shown in Figure 6. The description of the variants of the first and second embodiments are also relevant to this variant of the third embodiment.

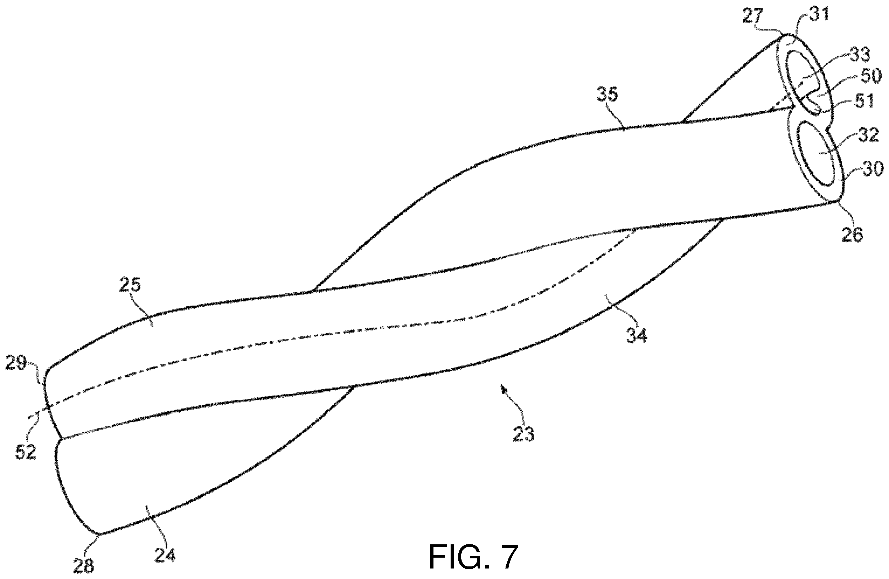

Referring to Figure 7, the third embodiment is shown in its final form in which there is located around the inner surface 51 of the tubular perimeter wall 31 an axially extending, internal helical protrusion 50, which projects radially inwardly into the outflow lumen 33. The helix angle of the axially extending internal helical protrusion 50 is 20°. In this embodiment, the cross-section of the axially extending internal helical protrusion 50 perpendicular to the longitudinal axis 52 of the tubular conduit 25 is of a bell shape. The axially extending internal helical protrusion 50 extends between the proximal and distal ends 27, 29 of the second tubular conduit 25 and consists of one single revolution. That is to say, the axially extending internal helical protrusion 50 makes one complete turn of 360° between the proximal and distal ends 27, 29 of the second tubular conduit 25. In alternative embodiments, the axially extending internal helical protrusion 50 is either shorter or longer than one single revolution and may, for example, be between 50% and 150% of a single revolution. In the intermediate conduits described above and as shown in Figures 3 to 6, the internal helical protrusion shown in Figure 7 is provided in the outflow lumen 33 thereof.

In use of the third embodiment (depicted in Figures 3 to 7), the conduit arrangement 23 is comprised within a dialysis catheter (not shown) and the distal ends 28, 29 of the first and second tubular conduits 24, 25 are connected so as to be in fluid communication with a dialysis machine (not shown) and the proximal ends 26, 27 of the first and second tubular conduits 24, 25 are inserted into the vein of an individual who is suffering from kidney failure (not shown). The structure of the proximal and distal ends 26, 27 of the conduit arrangement 23 can be analogous to that described above in relation to the first embodiment.

Once the distal ends 28, 29 of the first and second tubular conduits 24, 25 are connected so as to be in fluid communication with the dialysis machine and the proximal ends 26, 27 are inserted into the vein of the individual, blood flows independently from the individual to the dialysis machine through the inflow lumen 32 of the first tubular conduit 24 and from the dialysis machine to the individual through the outflow lumen 33 of the second tubular conduit 25. As the blood passes through the two lumens 32, 33 the helical pathway of said lumens imparts helical flow on the blood, which reduces turbulence in the blood. The helical blood flow continues after the blood exits the outflow lumen 33 and enters the blood vessel of the individual. Thus turbulent blood flow is reduced, or even eliminated, in the blood vessel downstream of the outflow lumen 33.

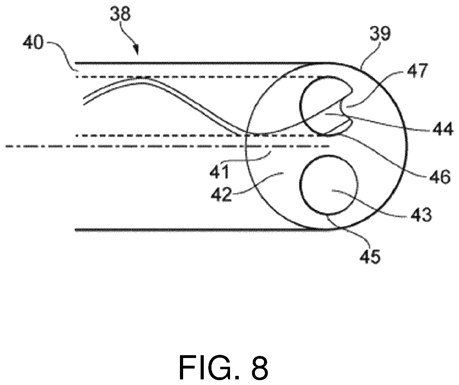

Referring to Figure 8, a portion of a tubular conduit 38 in accordance with a fourth embodiment of the present invention is shown. The tubular conduit 38 comprises proximal and distal ends 39, 40 and a longitudinal axis therebetween 41. The tubular conduit 38 is defined by an axially extending tubular wall 42. Thus, the tubular conduit 38 has a circular cross-section. The tubular conduit further comprises two axially extending tubular lumens 43, 44: an inflow lumen 43 and an outflow lumen 44. Each of the inflow and outflow lumens 43, 44 is defined by an inner surface 45, 46 of the axially extending tubular wall 42 and each of the inflow and outflow lumens 43, 44 is substantially circular in cross-section. The inflow and outflow lumens 43, 44 are located side-by-side within the tubular conduit 38 and each of said lumens follows a pathway which is substantially straight with respect to the longitudinal axis 41 of the tubular conduit 38, subject to any overall curvature of the tubular conduit 38. Each of the inflow and outflow lumens 43, 44 independently permits fluid communication between the proximal and distal ends 39, 40 of the tubular conduit 38. Located around the inner surface 46 of the axially extending tubular wall 42 which defines the outflow lumen 44 is an axially extending internal helical protrusion 47, which projects radially inwardly into the outflow lumen 44. The helix angle of the axially extending internal helical protrusion 47 is 20°. In this embodiment, the cross-section of the axially extending internal helical protrusion 47 perpendicular to the longitudinal axis 41 of the tubular conduit 38 is of a bell shape. The axially extending internal helical protrusion 47 extends between the proximal and distal ends 39, 40 of the tubular conduit 38 and consists of one single revolution. That is to say, the axially extending internal helical protrusion 47 makes one complete turn of 360° between the proximal and distal ends 39, 40 of the tubular conduit 38. In alternative embodiments, the axially extending internal helical protrusion 47 is either shorter or longer than one single revolution and may, for example, be between 50% and 150% of a single revolution.

In a variant of the fourth embodiment, the axially extending internal helical protrusion 47 extends for only a portion of the length of the tubular conduit 38. The description of the variants of the first and second embodiments are also relevant to this variant of the fourth embodiment.

In use, the distal end 40 of the tubular conduit 38 is connected so as to be in fluid communication with a dialysis machine (not shown) and the proximal end 39 of the tubular conduit 38 is inserted into the vein of an individual who is suffering from kidney failure (not shown). The structure of the proximal and distal ends 39, 40 of the tubular conduit 38 can be analogous to that described above in relation to the first embodiment.

Once the distal end 40 of the tubular conduit 38 is connected so as to be in fluid communication with the dialysis machine and the proximal end 39 is inserted into the vein of the individual, blood flows independently from the individual to the dialysis machine through the inflow lumen 43 of the tubular conduit 38 and from the dialysis machine to the individual through the outflow lumen 44 of the tubular conduit 38. As the blood passes the axially extending internal helical protrusion 47 of the outflow lumen 44, helical flow is imparted on the blood, which reduces turbulence in the blood. Furthermore, the helical blood flow continues after the blood exits the outflow lumen 44 and enters the blood vessel of the individual. Thus turbulent blood flow is reduced, or even eliminated, in the blood vessel downstream of the outflow lumen 44.

In one embodiment, the tubular conduit 1, 1', 13, 24, 25, 38 is made from polyurethane. However, in alternative embodiments, the tubular conduit 1, 1', 13, 24, 25, 38 is made from PVC, PTFE, latex, silicone or carbothane. The interior of each of the inflow and outflow lumens 10, 11, 10', 11', 18, 20, 32, 33, 43, 44 is made from a biocompatible material. In the embodiments described above, the tubular conduit 1, 1', 13, 24, 25, 38 is flexible. The conduit arrangement 23 of the third embodiment is also flexible.

In the embodiments described above, the helical flow imparted on the blood in the tubular lumen is spiral laminar flow such that natural blood flow patterns are replicated. Spiral laminar flow has been observed *in vivo* in both animals and humans and may be a constant (veins primarily) or pulsatile (arteries) flow waveform. In the tubular lumen of the embodiments of the present invention, spiral laminar flow means that in addition to the laminar flow in the direction of the tubular conduit, there is a rotation in the plane of the cross-section of the tubular conduit, produced by the portion of the tubular lumen that is capable of imparting helical flow.

If the tubular conduit of any of the embodiments described above were to comprise a narrowed section or a bifurcation or branch, spiral laminar flow would be maintained through the narrowing of a tubular conduit and beyond the branches or bifurcations of the tubular conduit.

In the embodiments described above, the cross-section of the axially extending internal helical protrusion 12, 21, 47, 50 perpendicular to the longitudinal axis 4', 16, 41, 52 of the tubular conduit 1', 13, 38, 25 is of a symmetric bell shape. However, in variants of these embodiments, the axially extending internal helical protrusion 12, 21, 47, 50 instead has an asymmetrical cross-section.

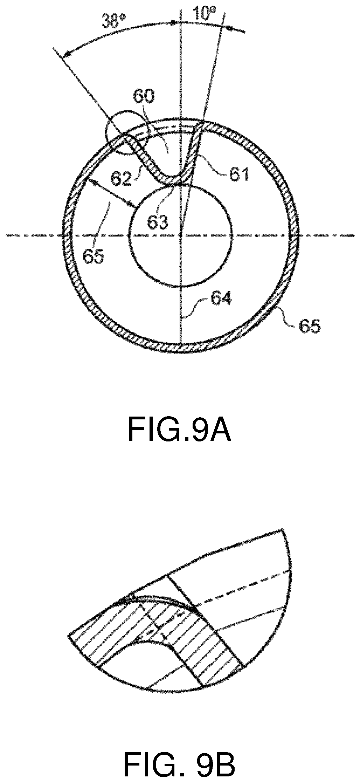

Referring to Figure 9, a cross-section of an axially extending internal helical protrusion 60 with an asymmetric profile is shown. As shown in Figure 9, the axially extending internal helical protrusion has a first face 61 and a second face 62 coupled together by a curved surface 63. The main central section of the first face 61 is at an angle of approximately 10° to a diameter 64 of the tubular conduit 65 that intersects the curved surface 63. The main central section of the second face 62 is at an angle of approximately 38° to a diameter 64 of the tubular conduit 65 that intersects the curved surface 63. The height 65 of the axially extending internal helical protrusion 60 is half the radius of the tubular conduit 65. In variants of the second, third, and fourth embodiments of the present invention described above, an axially extending internal helical protrusion 60 having an asymmetric cross-section, as shown in Figure 9, is provided.

In use, the tubular conduit 65 comprising the axially extending internal helical protrusion 60 with the asymmetric profile is orientated so that the blood flow is against the first face 61.

In alternative embodiments, the axially extending internal helical protrusion 12, 21, 47, 50 has a different cross-section perpendicular to the longitudinal axis 4', 16, 41, 52 of the tubular conduit 1', 13, 38, 25, instead of a bell shape, such as having a U-shaped cross-section as is described in WO03/045279 or a triangular cross-section such as described in WO2005/004751.

In the embodiments described above, the helix angle of the helical pathway or the axially extending internal helical protrusion 12, 21, 47, 50 is 20°. However, in other embodiments the helical pathway or the axially extending internal helical protrusion 12, 21, 47, 50 has a helix angle of between 5° and 50°, 5° and 40°, 5° and 30°, 5° and 25° and preferably between 5° and 20°, more preferably between 8° and 20°.

In the embodiments described above, the axially extended internal helical protrusion 12, 21, 47 is comprised within a portion of the dialysis catheter that is not implanted into the body of an individual. However, in alternative embodiments, the feature is comprised within a portion of the dialysis catheter that is implanted into the body of an individual.

The first embodiment described above is manufactured using the following method. The tubular conduit 1 is extruded using standard techniques known in the art. The portion of the tubular conduit 1 is which the inflow and outflow lumens 10, 1 1 follow a helical pathway is manufactured through use of a rotating extrusion die during the extrusion process. In an alternative method of manufacture, the tubular conduit 1 is extruded such that the inflow and outflow lumens 10, 11 follow a substantially straight pathway. The helical pathway of the inflow and outflow lumens 10, 11 is then introduced post-extrusion using appropriate tools and temperature.

The third embodiment described above is manufactured using a similar method as for the first embodiment. The first and second tubular conduits 24, 25 are extruded using standard techniques known in the art. The first and second tubular conduits 24, 25 are intertwined such that the inflow and outflow lumens 32, 33 independently follow a helical pathway through use of a rotating extrusion die during the extrusion process. In an alternative method of manufacture, the first and second tubular conduits 24, 25 are extruded such that the inflow and outflow lumens 32, 33 follow a substantially straight pathway. The first and second tubular conduits 24, 25 are then intertwined post- extrusion using appropriate tools and temperature.

The axially extending internal helical protrusion 12, 21, 47 is produced by injection moulding and is then connected to rest of the tubular conduit 1', 13, 38 by using thermal or radio frequency (RF) fusion or by using solvent bonding or gluing. The tubular conduit 1', 13, 38 minus the axially extending internal helical protrusion is made using standard techniques in the field. The axially extending internal helical protrusion 12, 21, 47 is made from the same material as the rest of the tubular conduit 1', 13, 38.

Dual or multi-lumen catheters are used for various medical applications, aside from dialysis catheters, and may transfer fluids other than blood independently to and from an individual.

## Claims

1. An intravenous dialysis catheter (1,1',13,23,38) comprising distal (3,15,28,29,40) and proximal (2,14,26,27,39) ends and two tubular lumens (10,10',18,32,43; 11,11',20,33,44), wherein each tubular lumen (10,10',18,32,43; 11,11',20,33,44) comprises a longitudinal axis (4,4',16,41,52) extending between the distal (3,15,28,29,40) and proximal (2,14,26,27,39) ends and independently permits fluid communication between the distal (3,15,28,29,40) and proximal ends (2,14,26,27,39) and wherein the interior of each of the two tubular lumens (10,10',18,32,43; 11,11',20,33,44) is made from a biocompatible material,
wherein at least a portion of at least one tubular lumen (10,10',18,32,43; 11,11 ',20,33,44) comprises an axially extending internal helical protrusion (12, 21, 47) located around the inside of the tubular lumen (10,10',18,32,43; 11,11',20,33,44) for imparting helical flow on fluid passing through said portion of the tubular lumen (10,10',18,32,43; 11,11',20,33,44) and wherein said helical flow is spiral laminar flow, and
**characterised in that** the catheter (1,1',13,23,38) comprises a tubular conduit and a septum (6,6') which divides the tubular conduit parallel to its longitudinal axis (4,4',16,41,52) so as to define the two tubular lumens (10,10',18,32,43; 11,11',20,33,44).

2. The catheter (1,1',13,23,38) according to claim 1, wherein the septum (6,6') extends substantially across a diameter of the tubular conduit.

3. The catheter (1,1',13,23,38) according to claim 1, wherein the catheter (1,1',13,23,38) comprises a tubular conduit and wherein the two tubular lumens (10,10',18,32,43; 11,11',20,33,44) are located side-by-side in said tubular conduit.

4. The catheter (1,1',13,23,38) according to any one of the preceding claims, wherein the pathway of each of the two tubular lumens (10,10',18,32,43; 11,11',20,33,44) is straight with respect to the longitudinal axis of each tubular lumen (10,10',18,32,43; 11,11 ',20,33,44).

5. The catheter (1,1',13,23,38) according to either one of claims 1 and 2, wherein said portion of the tubular lumen (10,10',18,32,43; 11,11',20,33,44) follows a helical pathway.

6. The catheter (1,1',13,23,38) according to either one of claims 1 and 2, wherein sequential non-circular cross-sections of said portion of the tubular lumen (10,10',18,32,43; 11,11 ',20,33,44) transition rotationally along the longitudinal axis of the tubular lumen (10,10',18,32,43; 11,11 ',20,33,44).

7. The catheter (1,1',13,23,38) according to claim 1, wherein the catheter (1,1',13,23,38) comprises first and second tubular conduits (24,25) fused together, one of the tubular lumens (32,33) being located in the first tubular conduit (24) and the other of the tubular lumens (32,33) being located in the second tubular conduit (25).

8. The catheter (1,1',13,23,38) according to claim 7, wherein each of the two tubular lumens (32,33) independently follows a helical pathway.

## Patentansprüche

1. Intravenöser Dialysekatheter (1, 1', 13,23,38), der distale (3, 15,28,29,40) und proximale (2, 14,26,27,39) Enden und zwei röhrenförmige Lumen (10, 10', 18,32,43; 11, 11', 20,33,44) umfasst, wobei jedes röhrenförmige Lumen (10, 10', 18,32,43; 11, 11',20,33,44) eine Längsachse (4,4', 16,41 ,52) umfasst, die sich zwischen den distalen (3, 15,28,29,40) und proximalen (2,14,26,27,39) Enden erstreckt und unabhängig eine Fluidkommunikation zwischen den distalen (3, 15,28,29,40) und proximalen Enden (2, 14,26,27,39) ermöglicht, und wobei das Innere jedes der zwei röhrenförmigen Lumen (10, 10', 18,32,43; 11, 11',20,33,44) aus einem biokompatiblen Material besteht,
wobei mindestens ein Abschnitt von mindestens einem röhrenförmigen Lumen (10, 10', 18,32,43; 11, 11',20,33,44) einen sich axial erstreckenden inneren spiralförmigen Vorsprung (12,21, 47) umfasst, der um das Innere des röhrenförmigen Lumens (10, 10', 18,32,43; 11, 11',20,33,44) angeordnet ist, um dem durch den Abschnitt des röhrenförmigen Lumens (10, 10', 18,32,43; 11, 11',20,33,44) fließenden Fluid eine spiralförmige Strömung zu verleihen und wobei die spiralförmige Strömung eine spiralförmige laminare Strömung ist, und
**dadurch gekennzeichnet, dass**
der Katheter (1, 1',13,23,38) eine röhrenförmige Leitung und ein Septum (6,6') umfasst, das die röhrenförmige Leitung parallel zu ihrer Längsachse (4,4', 16,41,52) teilt, um die zwei röhrenförmigen Lumen (10, 10', 18,32,43; 11, 11',20,33,44) zu definieren.

2. Katheter (1, 1', 13,23,38) nach Anspruch 1, wobei sich das Septum (6,6') im Wesentlichen über einen Durchmesser der röhrenförmigen Leitung erstreckt.

3. Katheter (1, 1', 13,23,38) nach Anspruch 1, wobei der Katheter (1, 1', 13,23,38) eine röhrenförmige Leitung umfasst und wobei die zwei röhrenförmigen Lumen (10, 10', 18,32,43; 11, 11', 20,33,44) nebeneinander in der röhrenförmigen Leitung angeordnet sind.

4. Katheter (1, 1',13,23,38) nach einem der vorhergehenden Ansprüche, wobei der Weg jedes der zwei röhrenförmigen Lumen (10, 10', 18,32,43; 11, 11',20,33,44) in Bezug auf die Längsachse jedes röhrenförmigen Lumens (10, 10', 18,32,43; 11, 11',20,33,44) gerade ist.

5. Katheter (1, 1', 13,23,38) nach einem der Ansprüche 1 und 2, wobei der Abschnitt des röhrenförmigen Lumens (10, 10', 18,32,43; 11, 11', 20,33,44) einem helikalen Weg folgt.

6. Katheter (1, 1', 13,23,38) nach einem der Ansprüche 1 und 2, wobei aufeinanderfolgende nicht kreisförmige Querschnitte des Abschnitts des röhrenförmigen Lumens (10, 10', 18,32,43; 11, 11',20,33,44) drehbar entlang der Längsachse des röhrenförmigen Lumens (10, 10', 18,32,43; 11, 11',20,33,44) übergehen.

7. Katheter (1, 1', 13,23,38) nach Anspruch 1, wobei der Katheter (1, 1', 13,23,38) eine erste und zweite röhrenförmige Leitung (24,25) umfasst, die miteinander verschmolzen sind, wobei eines der röhrenförmigen Lumen (32,33) in der ersten röhrenförmigen Leitung (24) angeordnet ist und das andere der röhrenförmigen Lumen (32,33) in der zweiten röhrenförmigen Leitung (25) angeordnet ist.

8. Katheter (1, 1', 13,23,38) nach Anspruch 7, wobei jedes der zwei röhrenförmigen Lumen (32,33) unabhängig voneinander einem spiralförmigen Weg folgt.

## Revendications

1. Cathéter de dialyse intraveineuse (1, 1', 13, 23, 38) comprenant des extrémités distales (3, 15, 28, 29, 40) et proximales (2, 14, 26, 27, 39) et deux lumières tubulaires (10, 10', 18, 32, 43 ; 11, 11', 20, 33, 44), chaque lumière tubulaire (10, 10', 18, 32, 43 ; 11, 11', 20, 33, 44) comprenant un axe longitudinal (4, 4', 16, 41, 52) s'étendant entre les extrémités distales (3, 15, 28, 29, 40) et proximales (2, 14, 26, 27, 39) et permettant indépendamment une communication fluidique entre les extrémités distales (3, 15, 28, 29, 40) et proximales (2, 14, 26, 27, 39) et l'intérieur de chacune des deux lumières tubulaires (10, 10', 18, 32, 43 ; 11, 11', 20, 33, 44) étant constitué d'un matériau biocompatible,
au moins une partie d'au moins une lumière tubulaire (10, 10', 18, 32, 43, 11, 11', 20, 33, 44) comprenant une saillie hélicoïdale interne s'étendant axialement (12, 21, 47) située autour de l'intérieur de la lumière tubulaire (10, 10', 18, 32, 43 ; 11, 11', 20, 33, 44) pour transmettre un écoulement hélicoïdal au fluide passant à travers ladite partie de la lumière tubulaire (10, 10', 18, 32, 43 ; 11, 11', 20, 33, 44) et ledit écoulement hélicoïdal étant un écoulement laminaire en spirale, et
**caractérisé en ce que**
le cathéter (1, 1', 13, 23, 38) comprend un conduit tubulaire et un septum (6, 6') qui divise le conduit tubulaire parallèlement à son axe longitudinal (4, 4', 16, 41, 52) de manière à définir les deux lumières tubulaires (10, 10', 18, 32, 43 ; 11, 11', 20, 33, 44).

2. Cathéter (1, 1', 13, 23, 38) selon la revendication 1, ledit septum (6, 6') s'étendant sensiblement sur un diamètre du conduit tubulaire.

3. Cathéter (1, 1', 13, 23, 38) selon la revendication 1, ledit cathéter (1, 1', 13, 23, 38) comprenant un conduit tubulaire et lesdites deux lumières tubulaires (10, 10', 18, 32, 43 ; 11, 11', 20, 33, 44) étant situés côte à côte dans ledit conduit tubulaire.

4. Cathéter (1, 1', 13, 23, 38) selon l'une quelconque des revendications précédentes, le trajet de chacune des deux lumières tubulaires (10, 10', 18, 32, 43 ; 11, 11', 20, 33, 44) étant droit par rapport à l'axe longitudinal de chaque lumière tubulaire (10, 10', 18, 32, 43 ; 11, 11', 20, 33, 44).

5. Cathéter (1, 1', 13, 23, 38) selon l'une quelconque des revendications 1 et 2, ladite partie de la lumière tubulaire (10, 10', 18, 32, 43 ; 11, 11', 20, 33, 44) suivant un trajet hélicoïdal.

6. Cathéter (1, 1', 13, 23, 38) selon l'une quelconque des revendications 1 et 2, des sections transversales non circulaires séquentielles de ladite partie de la lumière tubulaire (10, 10', 18, 32, 43 ; 11, 11', 20, 33, 44) passant en rotation le long de l'axe longitudinal de la lumière tubulaire (10, 10', 18, 32, 43 ; 11, 11', 20, 33, 44).

7. Cathéter (1, 1', 13, 23, 38) selon la revendication 1, ledit cathéter (1, 1', 13, 23, 38) comprenant des premier et second conduits tubulaires (24, 25) fusionnés ensemble, l'une des lumières tubulaires (32, 33) étant situées dans le premier conduit tubulaire (24) et l'autre des lumières tubulaires (32, 33) étant située dans le second conduit tubulaire (25).

8. Cathéter (1, 1', 13, 23, 38) selon la revendication 7, chacune des deux lumières tubulaires (32, 33) suivant indépendamment un trajet hélicoïdal.
